# EUROPÄISCHE PATENTSCHRIFT

(11) **EP 2 347 006 B1**
(45) Veröffentlichungstag und Bekanntmachung des Hinweises auf die Patenterteilung: **12.09.2012**
(21) Anmeldenummer: 09749102.1
(22) Anmeldetag: 04.11.2009
(51) Int. Cl.: C12Q 1/02, G01N 33/46, G01N 17/00

(54) **VERFAHREN ZUR QUALITÄTSBESTIMMUNG VON ZUR DACHEINDECKUNG VORGESEHENEM REET SOWIE ZUR PRÜFUNG VON VERFAHREN ZUM PROPHYLAKTISCHEN REETSCHUTZ**
METHODS FOR DETERMINING THE QUALITY OF REEDS PROVIDED FOR ROOFING, AND ALSO FOR TESTING METHODS FOR PREVENTIVE REED PROTECTION
PROCÉDÉ DE DÉTERMINATION DE LA QUALITÉ D' UN CHAUME PRÉVU POUR LA TOITURE ET DE VÉRIFICATION DE PROCÉDÉS DE PROTECTION PROPHYLACTIQUE D' UN CHAUME

(30) Priorität: 05.11.2008 DE 102008056486; 17.02.2009 DE 102009000931
(43) Veröffentlichungstag der Anmeldung: 27.07.2011
(73) Patentinhaber: Ernst-Moritz-Arndt-Universität Greifswald, 17487 Greifswald (DE)
(72) Erfinder: SCHAUER, Frieder, 17509 Lubmin (DE); JÜLICH, Wolf-Dieter, 17489 Greifswald (DE); KREISEL, Hanns, 17498 Weitenhagen OT Potthagen (DE); LINDEQUIST, Ulrike, 17491 Greifswald (DE); HOFMANN, Klaus, 17498 Klein Petershagen (DE)
(74) Vertreter: Gulde Hengelhaupt Ziebig & Schneider
(86) Internationale Anmeldenummer: PCT/EP2009/064614
(87) Internationale Veröffentlichungsnummer: WO 2010/052240

(56) Entgegenhaltungen:
- "Info 03/07 Qualitätssicherung von Reetdächern Erste Teilergebnisse der Forschung" März 2007 (2007-03), Seiten 1-3, XP007911602 Gefunden im Internet: URL:http://www.igbauernhaus.de/uploads/med ia/Newsletter_03-2007.pdf> [gefunden am 2010-02-08]
- "Einen Schritt weiter" 2007, XP8118542 Gefunden im Internet: URL:http://www.baufachinformation.de/zeits chriftenartikel.jsp?z=2007109018547>
- "Oben modert's" 2007, XP7911574 Gefunden im Internet: URL:http://www.zeit.de/2007/32/T-Reetdach? page=all&print=true>
- ANTHONY ET AL: "The macrofungi and decay of roofs thatched with water reed, Phragmites australis" MYCOLOGICAL RESEARCH, ELSEVIER, GB, Bd. 103, Nr. 10, 1. Oktober 1999 (1999-10-01), Seiten 1346-1352, XP022452089 ISSN: 0953-7562
- KREISEL HANNS ET AL: "[Crepidotus mollis on a reed-thatched roof.]" ZEITSCHRIFT FUER MYKOLOGIE, Bd. 68, Nr. 1, 2002, Seiten 41-44, XP8118533 ISSN: 0170-110X

## Beschreibung

### Technisches Gebiet

Die Erfindung betrifft Verfahren zur Qualitätsbestimmung von Reet und zur Prüfung von Verfahren zum prophylaktischen Reetschutz, sowie die Verwendung von ligninolytischen Pilzen in solchen Verfahren.

### Stand der Technik

Reet (auch: Reeth, Reith, Ried, Riet, Rohr) bezeichnet das Süßgras Schilfrohr (oder Schilf oder auch *Phragmites australis* bzw. *Phragmites communis),* welches bevorzugt an Ufern oder auf sumpfigem Gelände wächst. Reetmaterialien werden traditionell zur Dacheindeckung verwendet.

Mit Reet gedeckte Hausdächer stellen in den norddeutschen Ländern sowie in angrenzenden Staaten Nord- und Westeuropas einen prägenden Bestandteil der Kulturlandschaft dar, deren Erhalt sich deshalb in die Bemühungen zur Bewahrung der historischen Bausubstanz und der Umwelt einordnen lässt. Eine Bedrohung des Bestandes der Reetdächer durch fortschreitende Zerstörung des Reets stellt auch ein ernsthaftes Problem für den Fortbestand des traditionell und wirtschaftlich bedeutenden Reetdachdecker-Handwerks dar.

Die Ursachen des in den vergangenen 10 bis 15 Jahren verstärkt auftretenden Phänomens, dass neu eingedeckte Reetdächer nicht mehr die in der Vergangenheit beobachtete Lebensdauer von ca. 30 bis 50 Jahren erreichen, sondern häufig bereits innerhalb weniger Jahre total verrotten können, sind trotz intensiver Voruntersuchungen noch nicht abschließend geklärt.

Bei der Qualitätsbeurteilung von zur Dacheindeckung verwendetem Reet verlässt man sich bisher auf die Beurteilung durch den fachkundigen Reetdachdecker. Der Fachmann kann aus Erfahrung durch Anfassen, Fühlen, Brechen und nach der Farbe des Reets sagen, welches Material gut und welches schlecht ist.

Das für den Bau von Reetdächern verwendete Material sollte folgende Eigenschaften aufweisen (Quelle: Verein zur Förderung der Kulturlandschaft e.V.):
- Der Reethalm ist von gelblicher bis brauner Farbe.
- Die Oberflächenstruktur der Halme ist glatt und wasserabweisend.
- Die Halme haben einen leichten bis deutlichen Glanz.
- Das Halminnere ist hohl und sauber.
- Reethalme haben ein dichtes und starkes Festigungsgewebe.
- Halme weisen eine hohe Biege-, Bruch- und Druckfestigkeit auf.
- Die Halmlängen liegen zwischen 1,20 und 2,40 m.
- Die Halmdurchmesser liegen zwischen 0,3 und 0,9 cm.
- Die Reetbunde haben eine konische Form und einen Durchmesser von 60 cm.
- Das Reet wurde nach der Totreife geschnitten.
- Der Schnitt des Reets ist idealerweise nach einer Frostperiode erfolgt (Härtung des Halmes!).
- Die Reetbunde beinhalten keine Blattmasse und keine anderen Beimengungen.
- Die Halmbasis ist sauber, glatt geschnitten und nicht rissig.
- Das Reet ist nicht von Schadinsekten oder Pilzen befallen.
- Das Reet riecht neutral.
- Das Reet wurde bis zur Nutzung trocken und luftig gelagert.

Reet ist ein Naturprodukt. Demzufolge ist seine chemische Zusammensetzung von verschiedenen Faktoren abhängig und schwankt je nach Herkunft, nach Sorte und Aufwuchsbedingungen des Schilfs. Da zunehmend Importmaterialien verwendet werden, werden die Möglichkeiten der auf Erfahrung beruhenden Qualitätsprüfung weiter eingeschränkt.

Bei der Untersuchung der mikrobiellen Abbaubarkeit von Reet wurden verschiedene Mikroorganismen geprüft, die mit Schilfrohr assoziiert sind, so u.a. zellulolytische Bakterien, zellulolytische Pilze und Schimmelpilze, Flechten und Algen. Ein Qualitätskriterium konnte aus diesen Untersuchungen nicht abgeleitet werden.

Es ist bisher noch nicht gelungen, aus der chemischen Zusammensetzung des Reets einen zentralen Prüfparameter zu etablieren, mit dessen Hilfe die Beständigkeit gegen Verrottung vorhergesagt werden kann. Als erste Ansätze wurde eine Stickstoffbestimmung vorgenommen. Je höher der Stickstoffgehalt, desto schneller wird das Material bei Feuchtigkeit zersetzt. Je stickstoffärmer das Reet ist, desto besser ist die Qualität.

Zellulose ist mit einem Anteil von 42 bis 47 % die strukturbestimmende Komponente im Reet. Zelluloseketten lagern sich zu Milrofibrillen zusammen, die in eine Matrix vorwiegend aus Hemizellulosen (mit einem Anteil von etwa 23 bis 27 %) eingebaut sind und die hohe Zug- und Reißfestigkeit bedingen. Diese Struktur wird durch die Einlagerung von Lignin stabilisiert. Lignin ist ein amorpher, harzartiger und hochmolekularer Stoff, der in der Pflanze aus phenolischen Vorprodukten aufgebaut wird. Lignin ist im Reet mit einem Anteil von 10 bis 24 % vertreten und bewirkt durch Einlagerung in die Zellulosemembran eine Erhöhung der Druckfestigkeit und der Steifheit, verringert zugleich die Wasserwegsamkeit und erhöht die Widerstandsfähigkeit gegen mikrobiellen Abbau. In diese komplexe Struktur sind einerseits Wachse, Fette und Harze mit einem Anteil von 1,15 bis 1,17 % und andererseits Mineralstoffe, insbesondere Siliziumverbindungen mit einem Anteil von 4,7 bis 5,2 % eingebaut, die beide für die Widerstandsfähigkeit gegen mikrobiellen Abbau von Bedeutung sind. Insbesondere die fettähnlichen Substanzen bedingen ein geringes Wasseraufnahmevermögen.

Es hat nicht an Versuchen gefehlt, aus der chemischen Zusammensetzung des Reets Parameter für die Qualität abzuleiten und die Beständigkeit vorherzusagen. Da aber für die Beständigkeit gegen mikrobiellen Abbau die komplexe Struktur aus sehr verschiedenen Komponenten entscheidend ist, hat die Bestimmung einzelner Komponenten bisher nicht zum Erfolg geführt. Bei Erhaltung der komplexen Struktur sind auch im Prinzip relativ leicht abbaubare Komponenten wie die Hemizellulosen und die Zellulose vor dem mikrobiellen Abbau geschützt.

Zur Verbesserung der Beständigkeit von Reet werden verschiedene chemische und physikalische Verfahren der Vorbehandlung des Reets vorgeschlagen. Die Schwierigkeit bei der Bewertung von Methoden zum prophylaktischen Reetschutz besteht darin, dass es nach dem Stand nicht möglich ist, die Wirksamkeit dieser Methoden vorauszusagen. im Gegensatz zum prophylaktischen Holzschutz ist daher der prophylaktische Reetschutz wenig entwickelt.

Das Dokument "Info 03/07 Qualitätssicherung von Reetdächern, Erste Teilergebnisse der Forschung" März 2007 (2007-03) , Seiten 1-3", gefunden im Internet: URL:http://www.igbauernhaus.de/uploads/media/Newsletter-03-2007.pdf, gilt als nächster Stand der Technik. In ihm wird beschrieben, dass die Qualitätsbestimmung von Reet vom Fachmann durchgeführt werden kann, indem z.B. Biegefestigkeit, Druckfestigkeit, Wasseraufnahmefähigkeit usw. geprüft wird (siehe Seite 1, Absatz 2 oder Anmeldung, Seite 1, Abs. 5 bis Seite 2, Absatz 2). Weiterhin wird die Materialprüfung durch Nahinfrarotspektroskopie vorgeschlagen, um Ligninanteil, Stickstoffgehalt, usw. zu untersuchen (Seite 1, Absatz 4). Weiterhin wird eine mikroskopische Untersuchung der Reetqualität diskutiert (Seite 2, Absatz 4). Eine Untersuchung mit Hilfe ligninolytischer Pilze wird nicht vorgeschlagen.

Aufgabe der vorliegenden Erfindung ist es, einen oder mehrere Nachteile des geschilderten Standes der Technik zu vermindern oder ganz zu vermeiden. Insbesondere besteht die Aufgabe darin, einen Qualitätsparameter zu finden, der die komplexe Struktur dieser unterschiedlichen Komponenten berücksichtigt.

### Erfindungsgemäße Lösung

Die Aufgabe wird gelöst durch Bereitstellung eines Verfahrens zur Qualitätsbestimmung von Reet, dadurch gekennzeichnet, dass Reet-Material mit ligninolytischen Pilzen eines einzigen oder mehrerer verschiedener Stämme und/oder mit konditionierten Medien solcher Pilze oder Pilzgemische inkubiert wird.

Das erfindungsgemäße Verfahren kann insbesondere folgende Schritte umfassen:
a) Bereitstellung von Reet-Material;
b) Inkontaktbringen und Inkubation von Reet-Material mit ligninolytischen Pilzen eines einzigen oder mehrerer verschiedener Stämme und/oder mit konditionierten Medien solcher Pilze oder Pilzgemische;
c) Bestimmung von Veränderung des inkubierten Reet-Materials.

Es hat sich überraschenderweise herausgestellt, dass Cellulose und/oder Hemicellulose abbauende Bakterien und Pilze, die sehr zahlreich auf Reetdächern nachgewiesen werden können, das Reet nur nach einem vorangegangenen Lingninabbau durch ligninolytische Pilze effektiv zerstören können. Die lignolytischen Pilze, insbesondere Weißfäulepilze und streuzersetzende Pilze besitzen ein einzigartiges Enzymsystem, das außerhalb der Zelle (extrazellulär) wirksam ist und unspezifisch die aromatischen Strukturen des Lignins angreift. Die wichtigsten Komponenten des ligninolytischen Systems sind Peroxidasen (Mangan-Peroxidase, Lignin-Peroxidase) und Polyphenoloxidasen (Laccasen). Beide Enzymgruppen sind in der Lage, über Ein-Elektron-Transfer-Reaktionen, aus aromatischen Molekülen Radikale zu erzeugen. Dies hat die Destabilisierung des gesamten Ligninmoleküls zur Folge und führt über spontane Folgereaktionen zur Spaltung verschiedener chemischer Bindungen und damit zur Depolymerisation des Lignins. Die entstandenen niedermolekularen Ligninfragmente können durch die Pilze selbst oder durch andere Mikroorganismen aufgenommen und weiter metabolisiert werden. Das Cellulosegefüge des Reets bleibt dabei zunächst bestehen. Durch das starke Quellvermögen der Polysaccharide nimmt das Reet Feuchtigkeit auf und trocknet nicht mehr aus. Daher sind optimale Voraussetzungen für die Zersetzung der leichter abbaubaren Bestandteile des Reets durch andere Mikroorganismen entstanden. Im Gegensatz zu dem initialen Angriff durch die ligninolytischen Pilze sind die am Abbau von Cellulose und Hemizellulose beteiligten Enzyme wirkungs- und substratspezifisch, das heißt sie können nur einen bestimmten Stoff in einen bestimmten anderen überführen. Das Zusammenwirken des radikalisch wirkenden, extrazellulären Enzymsystems der ligninolytischen Pilze und der substratspezifischen Abbauleistungen der Bakterien und/oder zelluloseabbauenden Pilzen kann zu einem synergistischen Effekt bei der Reetzerstörung führen.

Mit dem erfindungsgemäßen Verfahren ist es nun möglich, die Vorraussetzungen für einen Initialangriff der Mikroorganismen zu beurteilen. Wenn das Reet in einem initialen Angriff nicht effektiv durch ligninolytische Pilze angegriffen werden kann, sind auch die leichter abbaubaren Bestandteile des Reets ausreichend geschützt. Das Reet hat eine höhere Qualität. Ist Lignin des Reets leicht effektiv durch ligninolytische Pilze abzubauen, so ist eine Entrittsmöglichkeit für z. B. Zellulose-abbauende Mikroorganismen geschaffen. Das Reet hat eine mindere Qualität. Es hat sich überraschenderweise herausgestellt, dass bei der erfindungsgemäßen Bestimmung der Veränderung, insbesondere der Abbaubarkeit von Reet-Material durch ligninolytische Pilze ein Leitparameter für eine hohe Reetqualität erhalten wird. Dieser Laborparameter kann durch die weiterhin nützliche visuelle Reetbeurteilung durch den Fachmann vorteilhaft ergänzt werden, da dieser Kriterien erfasst, die der visuellen Beurteilung nicht zugänglich sind. Die Bestimmung der Resistenz gegen ligninolytische Pilze unter festgelegten Bedingungen, gegebenenfalls in Verbindung mit der visuellen Reetbeurteilung, ist für sich genommen schon aussagekräftig. Trotzdem kann es zweckmäßig sein, weitere Kriterien wie Wasseraufnahmevermögen sowie Lignin- und Stickstoffgehalt separat zu bestimmen und zur Qualitätsbeurteilung mit heranzuziehen.

### Reet-Material:

Reet (auch: Reeth, Reith, Ried, Riet, Rohr) bezeichnet das Süßgras Schilfrohr (oder Schilf oder auch *Phragmites australis* bzw. *Phragmites communis*), welches bevorzugt an Ufern oder auf sumpfigem Gelände wächst. Unter Reet-Material wird für die Zwecke der vorliegenden Erfindung jedes Material verstanden, welches Teile der Reetpflanze enthält. Insbesondere können Wurzel, Rhizom, Halm, Stengel, Blatt, Blüte, Ähre und/oder Früchte verwendet werden. Dabei kann frisches Reet-Material zum Einsatz kommen oder Material in jedem gewünschten Trocknungsgrad. Da die Materialauswahl für ein Reetdach normalerweise an bereits einsatzfertig getrocknetem Reet durchgeführt wird, wird für das erfindungsgemäße Verfahren bevorzugt getrocknetes Reet-Material eingesetzt. Bevorzugt wird im erfindungsgemäßen Verfahren ein Halm verwendet oder Teile davon. Besonders bevorzugt kann Material aus dem wurzelnahen Halmbereich eingesetzt werden, ganz besonders bevorzugt werden Halmbereiche verwendet, die keine Internodien aufweisen. Grundsätzlich ist die Größe des verwendeten Halmbereichs nicht begrenzt und richtet sich im Allgemeinen nach den spezifischen praktischen Anforderungen der gewählten Ausführungsform des erfindungsgemäßen Verfahrens. Bevorzugt kommt Reet-Material zum Einsatz, welches aus einem Halmstück besteht mit einer Länge von 0,1 cm bis 50 cm, besonders bevorzugt mit einer Länge von 1,5 cm bis 15 cm, ganz besonders bevorzugt mit einer Länge von 2 cm bis 10 cm. Insbesondere kann das Reet-Material aus einem Halmstück mit einer Länge von 0,1 cm bis 50 cm Länge bestehen, bevorzugt mit einer Länge von 1,5 cm bis 15 cm, das weiter bevorzugt keine Internodien aufweist.

### Ligninolytische Pilze:

Erfindungsgemäß wird das Reet-Material mit ligninolytischen Pilzen eines einzigen oder mehrerer verschiedener Stämme inkubiert. Wesentlich für die Erfindung ist, dass die verwendeten Pilze oder Pilzstämme eine ligninolytische Aktivität aufweisen, also Lignin aus seinem pflanzlichen Kontext heraus abbauen können. Insbesondere können ligninolytische Pilze oder Pilzstämme eingesetzt werden, die Lignin auch oder sogar bevorzugt aus getrocknetem Pflanzenmaterial heraus abbauen können. Im erfindungsgemäßen Verfahren können bevorzugt ligninolytische Pilze oder Pilzstämme verwendet werden, die eine Laccase- und/oder Peroxidase-Aktivität aufweisen. Dabei wird die Aktivität der Laccase oder Peroxidasen im Kulturüberstand der zu testenden ligninolytischen Pilze oder Pilzstämme bestimmt. Geeignete Methoden zur Bestimmung der Laccase-Aktivität sind beschrieben in:
- Bourbonnais, R.L. and Paice, M.G. (1990). Oxidation of nonphenolic substrates: an expanded role for laccase in lignin biodegradation. FEBS Lett. 267, 99 - 102; und
- Roy-Arcand, L. and Archibald, F.S.J. (1991). Direct dechlorination of chlorophenolic compounds by laccase from Trametes (coriolus) versicolor. Enzyme Microbiol. Technol. 13, 194 - 203.

Es können ligninolytische Pilze oder Pilzstämme verwendet werden, die Laccase, aber im Wesentlichen keine Manganperoxidase-Aktivität aufweisen. Es können jedoch auch Pilze und Pilzgemische eingesetzt werden, bei denen die Laccase-Aktivität durch Peroxidasen-Aktivität, insbesondere durch Manganperoxidase-Aktivität und Ligninperoxidase-Aktivität ergänzt wird.

Ligninolytische Pilze oder Pilzstämme für die Verwendung im erfindungsgemäßen Verfahren können ausgewählt werden aus der Gruppe der Weißfäulepilze und Streu zersetzende Pilze und stammen bevorzugt aus den Gattungen Pycnoporus, Trametes, Coprinus, Geotrichum und/oder Mycena, insbesondere aus den Spezies Pycnoporus cinnabarinus, Trametes versicolor und/oder Coprinus domesticus. Die ausgewählten Weißfäulepilze sind auch in ihrer natürlichen Umgebung in der Regel als effektive Lignindezimierer bekannt.

### Konditionierte Medien:

Statt oder zusätzlich zu oben genannten ligninolytischen Pilzen oder Pilzstämmen können im erfindungsgemäßen Verfahren auch konditionierte Medien solcher Pilze oder Pilzgemische zur Inkubation mit Reet-Material verwendet werden. Unter dem Begriff "konditionierte Medien" werden für die Zwecke der Erfindung sowohl flüssige Überstände aus Kulturen von oben genannten ligninolytischen Pilzen oder Pilzstämmen verstanden, als auch Konzentrate und/oder Extrakte davon. Wesentlich im Sinne der Erfindung ist, dass diese konditionierten Medien eine Laccase-Aktivität gegebenenfalls in Kombination mit Peroxidase-Aktivität aufweisen.

### Inkubation:

Im erfindungsgemäßen Verfahren kann das Reet-Material mit ligninolytischen Pilzen eines einzigen oder mehrerer verschiedener Stämme inkubiert werden. Dies kann auf mindestens zwei unterschiedliche Arten und Weisen geschehen:
i) direktes Inkontaktbringen zwischen ligninolytischen Pilzen und zu untersuchendem Reet-Material. Das Inkontaktbringen findet dabei bevorzugt auf der Oberfläche des zu testenden Reet-Materials statt. Der Kontakt kann beispielsweise nur auf einem ausgewählten Teil der Oberfläche des Reet-Materials hergestellt werden oder über die gesamte Oberfläche des Reet-Materials. Der direkte Kontakt kann beispielsweise durch Beimpfen und/oder durch Besiedelung des Reet-Materials mit einer Probe von vermehrungsfähigen ligninolytischen Pilzen eines einzigen oder mehrerer verschiedener Stämme hergestellt werden.
ii) gemeinsame Inkubation von Reet-Material und ligninolytischem Pilzmaterial, ohne dass es zu einem direkten Kontakt kommt. Dabei sind die ligninolytischen Pilze und das zu untersuchende Reet-Material derart funktional angeordnet, dass es zu keinem physischen Kontakt zwischen beiden kommt, aber von den ligninolytischen Pilzen sezernierte extrazelluläre Enzyme mit ligninolytischer Aktivität, insbesondere mit Laccase-Aktivität, frei mit dem Reet-Material in direkten Kontakt gelangen können. Dabei kann es sich beispielsweise um eine Co-Kulturanordnung handeln, bei der eine ligninolytische Pilzkultur von dem zu untersuchenden Reet-Material physisch getrennt vorliegt, beispielsweise durch eine Membran oder ein sonstiges für Laccase-Aktivität durchlässiges Material oder Materialgemisch. Die ligninolytischen Pilze können auch eingeschlossen oder verkapselt eingesetzt werden, bevorzugt liegen die lig ninolytischen Pilze in Form von Gel-Einschluss-Verbindungen vor. Gel-Einschluss-Verbindungen von Weißfäule-Pilzen sind an sich bekannt und ihre Herstellung ist beispielhaft beschrieben in Chemie in unserer Zeit 32. 279 (2004). Weißfäulepilze können dabei mit einem an sich bekannten Verfahren in ein stabiles Gel überführt werden. Besonders vorteilhaft für eine Gel-Einschluss-Verbindung der Weißfäulepilze ist die Umhüllung mit Polyvinylalkohol. Dazu wird Polyvinylakohol mit der Pilzbiomasse und einem Vernetzungsmittel vermischt. Es entstehen Kugeln von 1-2 mm Durchmesser. In ihrem Innern kann der Pilz wachsen, die Exoenzyme produzieren und in die Umgebung entlassen, ist aber selbst vor einer Wechselwirkung mit der Umgebung und Umwelteinflüssen wie beispielsweise Bakterien, geschützt, da diese nicht in das gitterförmige Grundgerüst der Gel-Einschluss-Verbindungen eindringen können. Ein Vorteil der Inkubation von Reet-Material mit, ggf. in Einschlussverbindungen immobilisierten, ligninolytischen Pilzen eines einzigen oder mehrerer verschiedener Stämme ist, dass die ligninolytischen Pilze über die Inkubationsdauer hinweg in der Lage sind, ständig neue, unverbrauchte Laccase- und/oder Peroxidase-Aktivität bereitzustellen.

Durch die Verwendung von, ggf. in Einschlussverbindungen immobilisierten, ligninolytischen Pilzen ist es möglich, in den erfindungsgemäßen Verfahren, ligninolytische Pilzen und andere Mikroorganismen, wie beispielsweise Cellulose- und/oder Hemizellulose abbauende Bakterien und Pilze gleichzeitig auf das Reet einwirken zu lassen.

Ein weiterer Vorteil beim Einsatz von Gel-Einschluss-Verbindungen von ligninolytischen Pilzen ergibt sich aus der guten Lagerfähigkeit der Gel-Einschluss-Verbindungen. Daher ist es möglich, die Versuche über Monate mit einer Charge durchzuführen, was die Standardisierung erleichtert.

Im erfindungsgemäßen Verfahren kann das Reet-Material mit konditionierten Medien von ligninolytischen Pilzen eines einzigen oder mehrerer verschiedener Stämme inkubiert werden.

Um eine Veränderung im Reet-Material bestimmen zu können, wird das Reet-Material mit den ligninolytischen Pilzen und/oder mit den konditionierten Medien inkubiert. Die Inkubation findet dabei unter Bedingungen statt, die eine ligninolytische Aktivität zulassen. Insbesondere können Temperaturwerte, Feuchtigkeitswerte und/oder Gasversorgungsbedingungen so gewählt werden, dass eine ligninolytische Aktivität erhalten oder gefördert wird. Bevorzugt findet die Inkubation unter Bedingungen statt, die ein Wachstum mindestens eines Stammes der verwendeten ligninolytischen Pilze erlauben. Bevorzugt wird die Inkubation im erfindungsgemäßen Verfahren unter optimalen Wachstums- und/oder Enzymbildungsbedingungen mindestens eines Stammes der verwendeten ligninolytischen Pilze durchgeführt. Die Wahl von optimalen Inkubationsbedingungen hat den Vorteil, dass eine Veränderung des Reet-Materials so schnell wie möglich feststellbar ist und sich damit die Gesamtzeit für die Qualitätsbestimmung des Reets verkürzt. In einer besonderen Ausführungsform des erfindungsgemäßen Verfahrens wird die Inkubation über einen vorher festgelegten Zeitraum durchgeführt. Es ist ebenso möglich, die Zeit bis zur Erreichung eines vorher festgelegten Schwellenwertes der Reetschädigung zu messen. Die Wahl der exakten Inkubationsbedingungen hängt von den spezifischen, praktischen Anforderungen der gewählten Ausführungsform des erfindungsgemäßen Verfahrens ab. Neben der Wahl der ligninolytischen Pilze bzw. der konditionierten Medien, spielt beispielsweise auch die Größe des verwendeten Reet-Materials und die Dimensionierung des Inkubationsraumes eine Rolle. Der Fachmann ist ohne Schwierigkeiten in der Lage, für eine ausgewählte Ausführungsform des Verfahrens geeignete Inkubationsbedingungen zu wählen bzw. diese durch Routineversuche zu bestimmen. Geeignete Kulturbedingungen für ligninolytische Pilze sind dem Fachmann bekannt.

Das erfindungsgemäße Verfahren umfasst nicht nur Verfahren, bei denen das Reet-Material auf einem geeigneten Nährmedium, z. B. einem geeigneten Agar, aufliegt oder in diesen eingebettet ist und vorher oder anschließend mit ligninolytischen Pilzen inkubiert wird. Das erfindungsgemäße Verfahren bezieht auch solche Verfahren ein, bei denen das Reet-Material in einem geeigneten Gefäß, z. B. einem Eppendorf-Gefäß oder einem 15 ml Falcon-Röhrchen, mit einem konditionierten Medium in Kontakt gebracht und inkubiert wird.

Ein Vorteil der Verwendung von konditionierten Medien ist, dass eine Miniaturisierung leichter möglich ist und das Verfahren leichter standardisiert werden kann, da sich die Menge an eingesetzter ligninolytischer Aktivität leichter konstant halten lässt.

### Bestimmung von Veränderung des inkubierten Reet-Materials:

In einem zusätzlichen Schritt des erfindungsgemäßen Verfahrens wird eine Veränderung des inkubierten Reet-Materials bestimmt. Die Bestimmung der Veränderung des inkubierten Materials kann dabei in der Bestimmung jeder messbaren Eigenschaft des Reet-Materials bestehen, die durch einen Ligninabbau verändert werden kann. Es können beispielsweise Dichteund Festigkeitsmessungen durchgeführt werden, insbesondere eine Bestimmung der Zugfestigkeit, der Druckfestigkeit, der Biegefestigekeit, der Scherfestigkeit, der Spaltfestigkeit, der Torsionsfestigkeit und/oder der Härte.

Dabei können beispielsweise Verfahren angepasst und verwendet werden, die für die Prüfung von Holz auf Zerstörungen durch holzzerstörende Pilze entwickelt wurden.

In einer besonders einfachen zerstörungsfreien Ausführungsform wird der Eindringwiderstand eines Probekörpers beispielsweise einer Nadel gegen einen Federdruck gemessen. Dabei können kommerziell erhältliche Härtemessgeräte nach Shore (BAQ GmbH · Bienroder Weg 53 · 38108 Braunschweig) verwendet werden, die gegebenenfalls für die Prüfung der Reetqualität modifiziert werden.

Die Modifizierung kann vor allem darin bestehen, dass das Messgerät über eine geeignete Verknüpfung beispielsweise eine Luer-Luck-Verbindung mit sterilisierbaren Nadeln und/oder Prüfkörpern unterschiedlicher Geometrie ausgestattet wird, um eine Verschleppung der Pilze bzw. Bakterien zu verhindern. In einer bevorzugten Ausführungsform der erfindungsgemäßen Verfahren wird bei der Bestimmung von Veränderung des inkubierten Reet-Materials, insbesondere bei der Bestimmung einer Veränderung des Eindringwiderstandes, ein Härtemessgerät verwendet, dessen Prüfkörper sterilisierbar ist.

Die Veränderung kann beispielsweise als Unterschied zum Ausgangswert des Reet-Materials ausgedrückt werden (0-Wertvergleich), als Erreichen eines vorher festgelegten Schwellenwertes oder als ein Mehr oder Weniger an Veränderung im Vergleich zu einer Probe eines ausgewählten oder desselben Reet-Materials (Schwellenwertvergleich), welches unter denselben Bedingungen inkubiert wurde, allerdings ohne dass diese Probe mit ligninolytischen Pilzen und/oder mit konditionierten Medien in Kontakt gebracht worden ist (Referenzwertvergleich).

Insbesondere kann die Bestimmung von Veränderung des inkubierten Reet-Materials die Bestimmung einer Veränderung im Trockengewicht umfassen. Dabei wird beispielsweise nach erfolgter inkubation das inkubierte Reet-Material bis zur Gewichtskonstanz getrocknet und anschließend das Trockengewicht bestimmt.

Die Bestimmung von Veränderung des inkubierten Reet-Materials kann die Bestimmung einer Veränderung im Ligningehalt umfassen. Diese Veränderung kann beispielsweise durch Bestimmung des Ligningehalts im inkubierten Reet-Material gemessen werden, oder durch Bestimmung des Ligningehalts in einem Medium, welches während der Inkubation Kontakt zum zu testenden Reet-Material hatte.

Die Bestimmung von Veränderung des inkubierten Reet-Materials kann die Bestimmung einer Veränderung in der Dichte des inkubierten Reet-Materials oder eines Mediums, welches während der Inkubation Kontakt zum zu testenden Reet-Material hatte, beinhalten.

Die Bestimmung von Veränderung des inkubierten Reet-Materials kann die Bestimmung einer Veränderung in der Beständigkeit gegen eine Zugspannung umfassen. Insbesondere kann eine solche Veränderung dadurch bestimmt werden, dass vor oder zu Beginn der Inkubation an das Reet-Material eine vorher festgelegte Zugspannkraft angelegt wird und anschließend der Zeitpunkt bestimmt wird, zu dem das inkubierte Reet-Material der Zugspannkraft gerade nicht mehr standhält. Alternativ kann eine solche Veränderung dadurch bestimmt werden, dass nach der Inkubation an das inkubierte Reet-Material eine erste Zugspannkraft angelegt wird, die Zugspannkraft dann über die Zeit erhöht wird und die Zugspannkraft bestimmt wird, bei der das inkubierte Reet-Material der Zugspannung gerade nicht mehr standhält. Dabei kann die Zugspannkraft beispielsweise vermittelt werden durch Verwendung eines oder mehrerer Gewichte und/oder einer oder mehrerer Federn.

Im erfindungsgemäßen Verfahren können auch mehrere Verfahren und Parameter zur Bestimmung von Veränderung des inkubierten Reet-Materials zum Einsatz kommen.

Eine besondere Ausführungsform des erfindungsgemäßen Verfahrens umfasst die Schritte:
a) Bereitstellung von Reet-Material aus dem Halmbereich;
b) Inkontaktbringen und Inkubation von Reet-Material mit ligninolytischen Pilzen eines einzigen oder mehrerer verschiedener Stämme und/oder mit konditionierten Medien solcher Pilze oder Pilzgemische;
c) Bestimmung von Veränderung des inkubierten Reet-Materials durch Bestimmung einer Veränderung in der Beständigkeit gegen eine Zugspannung.

Das erfindungsgemäße Verfahren kann sich dadurch auszeichnen, dass das Reet-Material parallel und/oder anschließend in einem zusätzlichen Schritt mit Cellulose und/oder Hemicellulose abbauenden Bakterien und Pilzen inkubiert wird.

Das erfindungsgemäße Verfahren kann sich dadurch auszeichnen, dass zusätzlich der Ausgangsligningehalt, der Stickstoffgehalt und/oder die Wasseraufnahmefähigkeit des Reet-Materials vor und/oder nach der Inkubation bestimmt wird.

Daneben betrifft die vorliegende Erfindung auch ein Verfahren zur Prüfung von Verfahren zum prophylaktischen Reetschutz umfassend folgende Schritte:
a) Bereitstellung von Reet-Material;
b) Behandlung des Reet-Materials mit einem zu prüfenden Verfahren zum prophylaktischen Reetschutz,
c) Inkubation von Reet-Material mit ligninolytischen Pilzen eines einzigen oder mehrerer verschiedener Stämme und/oder mit konditionierten Medien solcher Pilze oder Pilzgemische;
d) Bestimmung von Veränderung des inkubierten Reet-Materials.

Das Verfahren zur Prüfung erlaubt es, anhand des beschriebenen Verfahrens zu bestimmen, ob eine ausgewählte Behandlung des Reet-Materials vor der Inkubation mit ligninolytischen Pilzen und/oder mit konditionierten Medien solcher Pilze dazu führt, dass eine Veränderung des inkubierten Reet-Materials verzögert, vermindert und/oder verhindert wird. Als Verfahren zum prophylaktischen Reetschutz kommen insbesondere solche chemischen und physikalischen Verfahren in Frage, die den Schutz von Reet zum Ziel haben. Ein Verfahren zum prophylaktischen Reetschutz kann beispielsweise eine Vorbehandlung des Reet-Materials mit einer ausgewählten Substanz oder einem ausgewählten Gemisch umfassen, oder eine Behandlung des Reet-Materials mit einer bestimmten Temperatur oder die Anwendung eines ausgewählten Trocknungsvorgangs umfassen.

Die Erfindung bezieht sich auch auf eine Verwendung von ligninolytischen Pilzen und/oder konditionierter Medien solcher Pilze oder Pilzgemische zur Qualitätsbestimmung von zur Dacheindeckung vorgesehenem Reet bzw. zur Prüfung von Verfahren zum prophylaktischen Reetschutz.

### Figuren

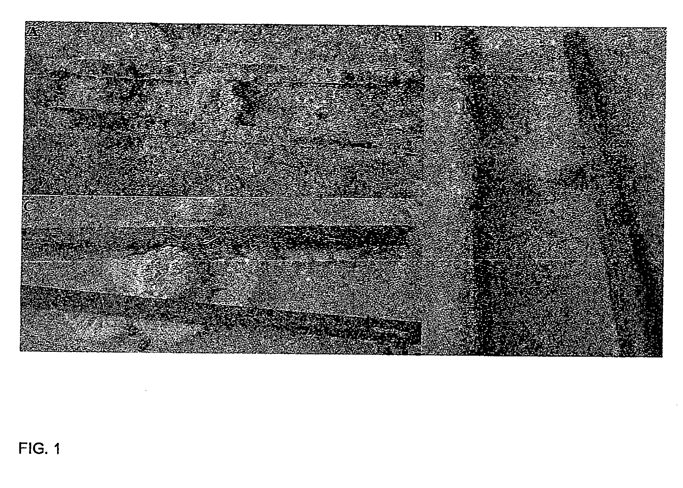

- FIG. 1: Bewuchs von Reetproben durch Mikroorganismen nach 45 Tagen Inkubation. A: *Pycnoporus cinnabarinus,B: Umbelopsis isabellina,* C: *Streptomyces spec.* Stamm 38 B

### Ausführungsbeispiele

### Beispiel 1: Abbau von Cellulose, Hemicellulosen und Lignin

Alle verwendeten Pilze wurden mit Hilfe von Agarplattenmethoden (De Koker et al., 2000) auf die Verwertung von Cellulose mittels Carboxymethylcelluloe (CMC) getestet, zusätzlich wurden die Stämme auf die Verwertung von Hemicellulosen mittels Xylan geprüft.

Zur Ermittlung ligninolytischer Aktivitäten der isolierten Pilzstämme wurde SMM-Festagar unter Zusatz von 2g/l Poly-R-478 verwendet (De Koker et al., 2000; Osma et al., 2007). Der purpurrote Farbstoff Poly-R-478 dient als Ligninmodell. Bei seiner Spaltung durch ligninolytische Pilzenzyme kommt es zur Entfärbung der Agarplatten.

Es wurden mehrere Pilze isoliert, die Poly-R-478 entfärben können: *Pycnoporus cinnabarinus* von einem stark verrotteten Dach in einem Waldgrundstück bei Darritz-Wahlendorf/Brandenburg. *P. cinnabarinus* ist ein Weißfäulepilz, der als guter Ligninabbauer bekannt ist.

*Geoirichum candidum* wurde von einer aus Ungarn stammenden in Wolfsee gelagerten Reetprobe isoliert (vgl. Tab. 1). Dieser Pilz ist in der Lage, Poly-R-478 sowie eine Reihe weiterer Verbindungen zu entfärben, die ähnliche Bindungen wie Lignin enthalten (Kim et al., 1995; Kim and Shoda, 1999). Ligninabbau durch diesen Pilz wurde bisher jedoch noch nicht nachgewiesen.

**Tabelle 1:**

| | **Abbau von** | | |
|---|---|---|---|
| **Wissenschaftlicher Name** | **CMC** | **Xylan** | **Poly-R-478** |
| *Pycnoporus cinnabarinus* | + | + | + |
| *Geotrichum candidium* | + | + | + |
| *Umbelopsis isabellina* | + | + | - |

### Beispiel 2: Nachweis ligninolytischer Enzymaktivitäten in ausgewählten Pilzstämmen

Zur Charakterisierung der Fähigkeiten zum Abbau des Reets wurden im Kulturüberstand ausgewählter Stämme die Aktivitäten der ligninolytischen Laccase, Mangan-peroxidase und der manganunabhängigen Peroxidase (Ligninperoxidase) gemessen. Die Laccase-Aktivität wurde sowohl mit 2,2-Azino-bis-(3-ethylbenzthiazoli-6-sulfonat) (ABTS) als auch mit α-Naphthol als Substrat nachgewiesen (Bourbonnais and Paice, 1990; Roy-Arcand and Archibald, 1991), die Manganperoxidase nach Wariishi et al. (1992), die mangan-unabhängige Peroxidase nach Roy-Arcand and Archibald (1991) sowie Peroxidasen mit Pyrogallol nach Kreisel und Schauer (1987). Die Ergebnisse der Enzymnachweise sind in Tabelle 2 aufgelistet.

Auffällig ist, dass der von uns isolierte Ligninabbauer *Pycnoporus cinnabarinus* 68 M ebenso wie der aus der Stammsammlung des Institutes für Mikrobiologie der Universität Greifswald stammende *P. cinnabarinus* SBUG 1044 nur über Laccase-Aktivität verfügt, nicht jedoch über Aktivitäten der Manganperoxidase und der Mn-unabhängigen Lignin-Peroxidase, die als stark Lignin abbauende Enzyme gelten. Der Laccase wurde in diesem Prozess vielfach nur eine sekundäre Rolle eingeräumt, obwohl inzwischen mehrere ligninolytische Pilze bekannt sind, in denen nur Laccase-Aktivität nachgewiesen wurde. Andere von uns isolierte Pilze wie *Geotrichum candidum, Coprinus domesticus* und *Umbelopsis isabellina* verfügen ebenfalls über α-Naphthol und/oder ABTS-abhängige Laccase-Aktivität, wobei im Falle von *Geotrichum candidum* Ligninabbau bisher nicht gezeigt wurde und die zwei anderen Pilze dazu unfähig sind.

Diese Methoden zur Enzymbestimmung stellen Testmethoden dar, mit deren Hilfe die Aktivitäten Reet bewohnender Pilze charakterisiert werden können. Sie liefern zusätzlich zu den Plattentests wertvolle Informationen zur Charakterisierung der Abbaufähigkeiten Reet zerstörender Pilze.

**Tabelle 2: Nachweis von Enzymen des Ligninabbaus in isolierten Pilzstämmen**

| Stamm | Aktivität (+/-) | | | | |
|---|---|---|---|---|---|
| | Laccase (ABTS) | Laccase (α-Naphthol) | Mangan-Peroxidase | Mn-unabh. Peroxidase | Peroxidase (Pyrogallol) |
| *Coprinus domesticus* 10/39 | - | + | - | - | (+) |
| *Geotrichum candidum* 10/43. 11/45, 15/58 | - | + | - | (+) | + |
| *Geotrichum candidum* 14/54 | + | + | - | - | + |
| *Umbelopsis isabellina 14*/*56* | - | + | - | - | - |
| *Pycnoporus cinnabarinus* 6/68 | + | + | - | - | (+) |
| *Pycnoporus cinnabarinus* SBUG 1044 | + | + | - | - | - |
| *Trametes versicolor* SBUG 1050 | + | + | (+) | + | + |

| | | | | | |
|---|---|---|---|---|---|
| -: keine Aktivität; +: Aktivitätsnachweis; (+): unklarer Aktivitätsnachweis | | | | | |

### Beispiel 3: Nachweis von Unterschieden im Abbau von Reetproben verschiedener Herkunft durch cellulolytische und ligninolytische Bakterien und Pilze

Durch die Inokulation steriler Reetproben verschiedener Herkunft (vgl. Tabelle 3) auf geeigneten Nährböden in Gegenwart cellulolytischer und ligninolytischer Bakterien bzw. Pilzen wurden verschiedene Probleme untersucht:
1. Können cellulolytische Bakterien und Pilze Reet effektiv angreifen oder sind dazu nur ligninolytische Organismen (Weißfäulepilze) in der Lage?
2. Werden Reetproben verschiedener Herkunft und Qualität unterschiedlich schnell angegriffen und ist dieser Test deshalb geeignet zur Bewertung der Reetqualität?
3. Welche Bedingungen sind für einen effektiven mikrobiellen Abbau des Reets notwendig?

Dabei wurde wie folgt verfahren:
Jeweils 7,5 cm lange Reetabschnitte aus dem unteren Halmbereich (ohne Internodien) wurden in nummerierte Reagenzgläser überführt und darin bis zur Gewichtskonstanz getrocknet (2 Stunden, 105°C). Das Gewicht dieser Proben wurde auf der Analysenwaage bestimmt. Die Reethalme wurden im Anschluss in den mit Wattestopfen verschlossenen Reagenzgläsern zweimal im Abstand von 24 Stunden im Dampftopf sterilisiert und waren damit bereit zur Untersuchung.

Als Testkeime für die Untersuchungen wurden folgende Organismen ausgewählt:
1. Cellulolytische Bakterien
   - Stamm 21 B
   - Stamm 38 B (*Streptomyces spec*.)
2. Der cellulolytische Pilz
   - *Umbelopsis isabellina* 1 M
3. Weißfäulepilze
   - *Pycnoporus cinnabarinus* 68 M
   - *Trametes versicolor* SBUG 1050 (Stammsammlung des Institutes für Mikrobiologie der Universität Greifswald)

**Tabelle 3: Herkunft der Reetproben für die Testung des mikrobiellen Abbaus**

| Probe/Herkunft | Lieferant | Bemerkungen |
|---|---|---|
| Deutschland Rügenscher Bodden | J.Paech, Samtens/Rügen | neue Ernte 2007/2008 |
| Deutschland Riemser Bodden | R.Carls, Neuenkirchen | neue Ernte 2007/2008 |
| Polen | R. Carls | neue Ernte 2007/2008 |
| Rumänien Donaudelta | B.Verch Rappin/Rügen | neue Ernte 2007/2008 |
| Türkei Schwarzes Meer | R. Carls | neue Ernte 2007/2008 |
| Südliches Afrika | R. Carls | kein Reet, "Savannengras Ernte 2006/2007 |

Die Bakterien wurden zur Gewinnung des Inokulums auf Nähragar bei Raumtemperatur vorkultiviert, für die Gewinnung des Inokulums der Pilzstämme wurde unter gleichen Bedingungen Malzagar genutzt.

Als Testmedien wurden eingesetzt:
1. Mineralsalz-Agarmedium K1 K2 (Schauer et al., 1980) mit reduziertem Glucosegehalt für die Testung der cellulolytischen Bakterien
2. SMM-Agar-Medium, ebenfalls mit reduziertem Glucosegehalt, für die Ansätze mit cellulolytischen und ligninolytischen Pilzen.

Die Testplatten wurden im Zentrum mit Agarstücken von 12 mm Durchmesser beimpft, die mittels sterilen Korkbohrers aus den Mikroorganismenrasen der Vorkulturen gewonnen wurden. Neben diese bewachsenen Agarplättchen wurden pro Platte jeweils zwei Probestücke Reet gleicher Herkunft gelegt. Als Kontrollplatten dienten unbeimpfte Ansätze mit Reetproben.

Anschließend wurden die Ansätze in feuchten Kammern 70 Tage bei Raumtemperatur (21 ± 1 °C) inkubiert. Parallelansätze wurden bereits nach 45 Tagen ausgewertet. Dazu wurden die Reetproben entnommen, von anhaftendem Pilzmycel bzw. Bakterienresten vorsichtig mechanisch gereinigt, anschließend bis zur Gewichtskonstanz getrocknet und erneut ausgewogen. Das Mittel der Trockengewichte beider Proben eines Ansatzes wurde zum Mittel der Ausgangsgewichte in Beziehung gesetzt (vgl. Tabelle 4).

Bereits nach 45 Tagen Inkubation waren die Ergebnisse sehr eindeutig. Erwartungsgemäß zeigten die unbeimpften Kontrollansätze praktisch keinen Masseverlust bei den eingesetzten Reetproben. Das Gleiche trifft auch auf die Ansätze mit den cellulolytischen Bakterienstämmen 21 B und 38 B, *Streptomyces spec.,* sowie den cellulolytischen Pilz *Umbelopsis isabellina* zu. Die Abnahme der Trockenmasse betrug in diesen Fällen maximal 3 % bei *Umbelopsis isabellina* und ist in den Ansätzen mit Bakterien noch geringer. Diese Werte liegen innerhalb der Fehlergrenze.

Dagegen konnte in den Ansätzen mit *Pycnoporus cinnabarinus* sowie *Trametes versicolor* ein deutlicher Masseverlust registriert werden, der bei *Pycnoporus cinnabarinus* zwischen 15 und 61 % und bei *Trametes versicolor* in Abhängigkeit von der Rohrcharge zwischen 32 und 55 % betrug.

Diese Organismen überwuchsen auch vollständig die Reetproben und drangen mit ihren Mycelien ca. bis 1 cm in die offenen Halmenden ein, während im Falle der cellulolytischen Bakterien und von *Umbelopsis isabellina* die Reetstücke bestenfalls geringfügig überwuchert wurden (Abb. 4). Der Masseverlust korreliert mit einer deutlichen Abnahme der Festigkeit der Reetproben. Sie verloren ihre Elastizität, wurden weich und zerbröselten leicht, während die Proben in den übrigen Ansätzen zwar durchfeuchtet waren, aber ihre Festigkeit bewahrt hatten.

Nach 70 Tagen Inkubation waren die Masseverluste in den Ansätzen mit Weißfäulepilzen noch deutlicher und erreichten immerhin 40 bis nahezu 80 % vom Ausgangsgewicht der Reetproben. Auch nach dieser relativ langen Inkubationszeit traten in den unbeimpften Kontrollansätzen keine Massenverluste auf, während in den Ansätzen mit den Bakterienstämmen 21 B und 38 B *(Streptomyces spec.)* sowie *Umbelopsis isabellina* bis auf eine Ausnahme diese Verluste deutlich unter 5 % lagen. Ob das überhaupt als Abbau gewertet werden kann, müssen weitere Untersuchungen zur statistischen Absicherung der Ergebnisse zeigen.

Diese Ergebnisse lassen folgende Schlussfolgerungen zu:
1. Offensichtlich sind nur Organismen mit ligninolytischen Aktivitäten, insbesondere Weißfäulepilze und/oder Streu zersetzende Pilze, in der Lage, das intakte Reet abzubauen. Bakterien und Pilze, die nur über cellulolytische und hemicellulolytische Aktivitäten verfügen, sind offenbar unfähig, Reet primär zu schädigen.
2. Der gewählte Versuchansatz ist grundsätzlich geeignet, Reetmaterial auf seine Widerstandsfähigkeit gegenüber mikrobiellen Angriffen zu testen. Mittels der verfügbaren Weißfäulepilze *Pycnoporus cinnabarinus, Trametes versicolor* u.a. kann die Abnahme der Biomasse über die Zeit unter Laborbedingungen untersucht werden, was Rückschlüsse auf die Reetqualität zulässt. Die vorliegenden Ergebnisse zeigen deutliche Unterschiede in den Abbauraten verschiedener Reetchargen.
3. Aussagen über den Einfluss von weiteren Milieufaktoren, die über Temperatur- und pH-Wert-Einflüsse hinausgehen (z. B. Wasserpotential, mineralische Nährstoffe, zusätzliche C-Quellen u.a.) auf die Abbaugeschwindigkeit des Reets sind nun auf solider Grundlage möglich geworden. Die gewählten Testbedingungen sind offensichtlich gut geeignet, um den Abbau der organischen Substanz durch die eingesetzten Pilze zu gewährleisten. Der Abbau des Reets im Labor erfolgte unter für die beteiligten Mikroorganismen nahezu optimalen Bedingungen, während sich Mikroorganismen auf Reetdächern mit ständig wechselnden, häufig sehr ungünstigen Bedingungen auseinandersetzen müssen und dort deshalb niemals auch nur annähernd diese Abbauraten des Reets erreicht werden.

**Tabelle 4: Mikrobieller Abbau von Reetproben verschiedener Herkunft (Bakterien)**

| Teststamm 1. Bakterien | Reetprobe/Herkunft | Trockengewichtsveränderung (%) | |
|---|---|---|---|
| | | 45 Tage | 70 Tage |
| 21 B | Deutschland Rügenscher Bodden | - 0,2±0,5 | -5,3±0,4 |
| | Deutschland Riemser Bodden | -0,3 ± 0,8 | -1,5 ± 3,0 |
| | Rumänien Donaudelta | -0,5 ± 0,5 | -4,9 ± 1,0 |
| | Polen | -0,2 ± 0,5 | -5,9 ± 2,4 |
| | Südliches Afrika Savannengras | -2,4 ± 0,3 | -1,1 ± 0,4 |
| | Türkei Schwarzmeerküste | -0,7 ± 0,8 | -1,7 ±0.3 |
| *Streptomyces spec.* 38B | Deutschland Rügenscher Bodden | -0,2 ± 0,5 | -8,0 ± 2,6 |
| | Deutschland Riemser Bodden | 0,0 | -1,5 ±1,4 |
| | Polen | 0,2 ± 0,9 | -2,0 ± 0,4 |
| | Südliches Afrika Savannengras | 1,0 ± 1,4 | -0,1 ± 0,1 |
| | Türkei Schwarzmeerküste | -1,7 ± 0,7 | -1,2 ± 0,6 |
| Kontrollen ohne Mikroorganismen | | -0,7 ± 2,1 | -1,6 ± 2,3 |

**Fortsetzung Tabelle 4 : Mikrobieller Abbau von Reetproben verschiedener Herkunft (Pilze)**

| Teststamm 2. Pilze | Reetprobe Herkunft | Trockengewichtsänderung (%) | |
|---|---|---|---|
| | | 45 Tage | 70 Tage |
| Umbelopsis isabellina | Deutschland Rügenscher Bodden | -3,0 ± 1,0 | -3,3 ± 0,4 |
| | Deutschland Riemser Bodden | -0,5 ± 0,3 | -0,1 ± 0,0 |
| | Rumänien Donaudelta | -1,4 ± 1,1 | -2,4 ± 0,8 |
| | Polen | -2,0 ± 1,3 | -0,5 ± 0 |
| | Türkei Schwarzmeerküste | -2,2 ± 0,5 | -0,3 ± 0 |
| *Pycnoporus cinnabarinus* | Deutschland Rügenscher Bodden | -25,7 ± 2,4 | -42,9 ± 1,4 |
| | Rumänien Donaudelta | -14,8 ± 2,9 | -37,1 ± 3,4 |
| | Südliches Afrika Savannengras | -29,0 ± 0,3 | -46,5 ± 5,2 |
| | Türkei Schwarzmeerküste | -45,8 ± 7,4 | -58,7 ± 4,6 |
| *Trametes versicolor* | Deutschland Rügenscher Bodden | -28,6 ± 1,1 | -71,7± 8,0 |
| | Deutschland Riemser Bodden | -31,8 ± 2,6 | -51,5 ± 2,4 |
| | Rumänien Donaudelta | -34,5 ± 4,4 | -50,0 ± 0,2 |
| | Polen | -55,4 ± 3,7 | -65,8 ± 5,1 |
| | Südliches Afrika Savannengras | -41,7 ± 3,7 | -76,5 ± 9,2 |

### Beispiel 4: Vergleich von zwei Reetproben verschiedener Herkunft bei Inkubation mit Pycnoporus cinnabarinus

Um eine signifikante Aussage über das Ausmaß des Abbaus von Reetmaterial durch ligninolytische Pilze machen zu können, wurden jeweils zehn Parallelproben von Reet von Rügen und aus Polen (vgl. Tab. 4) nach der oben beschriebenen Methode 35 Tage mit *Pycnoporus cinnabarinus* inkubiert und analysiert. Jeweils drei Parallel-Proben wurden als Kontrolle in unbeimpften Ansätzen mitgeführt.

Wie aus Tabelle 5 ersichtlich ist, blieb das Trockengewicht der unbeimpften Kontrollansätze wie erwartet durch die Inkubation von 35 Tagen unbeeinflusst, während in den Ansätzen mit *Pycnoporus cinnabarinus* deutliche Abnahmen ermittelt wurden.

**Tabelle 5: Abbau von Reetproben durch Pycnoporus cinnabarinus**

| Reetprobe | Abnahme nach 35 Tagen Inkubation (%) |
|---|---|
| Rügen/Bodden | -14,2 ± 8,5 |
| Rügen/ Bodden, Kontrolle | -0,5 ± 0,3 |
| Polen | -22,6 ± 7,8 |
| Polen, Kontrolle | -0,7 ± 0,3 |

| | |
|---|---|
| Kontrolle: Ansatz ohne Zusatz von Mikroorganismen | |

## Patentansprüche

1. Verfahren zur Qualitätsbestimmung von Reet,
**dadurch gekennzeichnet, dass**
Reet-Material mit ligninolytischen Pilzen eines einzigen oder mehrerer verschiedener Stämme und/oder mit konditionierten Medien solcher Pilze oder Pilzgemische inkubiert wird.

2. Verfahren nach Anspruch 1, umfassend die Schritte:
a) Bereitstellung von Reet-Material;
b) Inkubation von Reet-Material mit ligninolytischen Pilzen eines einzigen oder mehrerer verschiedener Stämme und/oder mit konditionierten Medien solcher Pilze oder Pilzgemische;
c) Bestimmung von Veränderungen des inkubierten Reet-Materials.

3. Verfahren zur Prüfung von Verfahren zum prophylaktischen Reetschutz umfassend folgende Schritte:
a) Bereitstellung von Reet-Material;
b) Behandlung des Reet-Materials mit einem zu prüfenden Verfahren zum prophylaktischen Reetschutz,
c) Inkubation von Reet-Material mit ligninolytischen Pilzen eines einzigen oder mehrerer verschiedener Stämme und/oder mit konditionierten Medien solcher Pilze oder Pilzgemische;
d) Bestimmung von Veränderungen des inkubierten Reet-Materials.

4. Verfahren nach einem der vorangegangenen Ansprüche,
**dadurch gekennzeichnet, dass**
das Reet-Material aus einem Halmstück mit einer Länge von 0,1 cm bis 50 cm Länge besteht, bevorzugt mit einer Länge von 1,5 cm bis 15 cm und das weiter bevorzugt aus dem wurzeinahen Halsbereich stammt und/oder keine Internodien aufweist.

5. Verfahren nach einem der vorangegangenen Ansprüche,
**dadurch gekennzeichnet, dass**
ligninolytische Pilze und/oder konditionierte Medien solcher Pilze oder Pilzgemische verwendet werden, die Laccase und/oder Peroxidase-Aktivität aufweisen.

6. Verfahren nach einem der vorangegangenen Ansprüche,
**dadurch gekennzeichnet, dass**
ligninolytische Pilze verwendet werden, die ausgewählt sind aus der Gruppe der Weißfäulepilze und/oder der streuzersetzenden Pilze, bevorzugt aus den Gattungen Pycnoporus, Trametes Coprinus, Geotrichum und/oder Mycena, insbesondere aus den Spezies Pycnoporus cinnabarinus, Trametes versicolor und/oder Coprinus domesticus und/oder konditionierte Medien solcher Pilze oder Pilzgemische zum Einsatz kommen.

7. Verfahren nach einem der vorangegangenen Ansprüche,
**dadurch gekennzeichnet, dass**
ligninolytische Pilze in Form von Gel-Einschluss-Verbindungen eingesetzt werden.

8. Verfahren nach einem der vorangegangenen Ansprüche,
**dadurch gekennzeichnet, dass**
die Bestimmung von Veränderung des inkubierten Reet-Materials die Bestimmung einer Veränderung im Trockengewicht, im Ligningehalt, in der Dichte, der Härte, im Endringwiderstand und/oder in der Beständigkeit umfasst.

9. Verfahren nach einem der vorangegangenen Ansprüche,
**dadurch gekennzeichnet, dass**
bei der Bestimmung von Veränderung des inkubierten Reet-Materials ein Härtemessgerät verwendet wird, dessen Prüfkörper sterilisierbar ist.

10. Verfahren nach einem der vorangegangenen Ansprüche,
**dadurch gekennzeichnet, dass**
das Reet-Material parallel und/oder anschließend in einem zusätzlichen Schritt mit Cellulose und/oder Hemicellulose abbauenden Bakterien und/oder Pilzen inkubiert wird.

11. Verfahren nach einem der vorangegangenen Ansprüche,
**dadurch gekennzeichnet, dass**
zusätzlich der Ausgangsligningehalt, der Stickstoffgehalt und/oder die Wasseraufnahmefähigkeit des Reet-Materials bestimmt wird.

12. Verwendung von ligninolytischen Pilzen und/oder konditionierter Medien solcher Pilze oder Pilzgemische zur Qualitätsbestimmung von zur Dacheindeckung vorgesehenem Reet.

13. Verwendung von ligninolytischen Pilzen und/oder konditionierter Medien solcher Pilze oder Pilzgemische zur Prüfung von Verfahren zum prophylaktischen Reetschutz.

## Claims

1. A method for determining the quality of reeds,
**characterized in that**
reed material is incubated with ligninolytic fungi of one single or several different strains and/or with conditioned media of such fungi or fungi mixtures.

2. A method according to claim 1, comprising the steps of:
a) providing reed material;
b) incubating reed material with ligninolytic fungi of one single or several different strains and/or with conditioned media of such fungi or fungi mixtures;
c) determining changes of the incubated reed material.

3. A method for testing methods for preventive reed protection, comprising the following steps:
a) providing reed material;
b) treating the reed material with a method for preventive reed protection to be tested;
c) incubating reed material with ligninolytic fungi of one single or several different strains and/or with conditioned media of such fungi or fungi mixtures;
d) determining changes of the incubated reed material.

4. A method according to any one of the preceding claims,
**characterized in that**
the reed material consists of a piece of stalk with a length of 0.1 cm to 50 cm, preferably with a length of 1.5 cm to 15 cm, and which further preferably stems from a stalk area near the root and/or has no internodes.

5. A method according to any one of the preceding claims,
**characterized in that**
ligninolytic fungi and/or conditioned media of such fungi or fungi mixtures are used, which have laccase and/or peroxidase activity.

6. A method according to any one of the preceding claims,
**characterized in that**
ligninolytic fungi are used, which are selected from the groups of white rot fungi and/or litter-decomposing fungi, preferably from the genera of Pycnoporus, Trametes Coprinus, Geotrichum and/or Mycena, in particular form the species of Pycnoporus cinnabarinus, Trametes vesicolor and/or Coprinus domesticus and/or conditioned media of such fungi or fungi mixtures.

7. A method according to any one of the preceding claims,
**characterized in that**
ligninolytic fungi in the form of gel inclusion compounds are used.

8. A method according to any one of the preceding claims,
**characterized in that**
determining change of the incubated reed material comprises determining a change in dry weight, lignin content, density, hardness, penetration resistance and/or durability.

9. A method according to any one of the preceding claims,
**characterized in that**
in determining change of the incubated reed material a durometer is used, the test body of which is sterilizable.

10. A method according to any one of the preceding claims,
**characterized in that**
the reed material is incubated with bacteria and/or fungi decomposing cellulose and/or hemicellulose in parallel and/or subsequently in an additional step.

11. A method according to any one of the preceding claims,
**characterized in that**
additionally, the initial lignin content, the nitrogen content and/or the water-absorbing capacity of the reed material is determined.

12. The use of ligninolytic fungi and/or conditioned media of such fungi or fungi mixtures for determining the quality of reed provided for roofing.

13. The use of ligninolytic fungi and/or conditioned media of such fungi or fungi mixtures for testing methods for preventive reed protection.

## Revendications

1. Procédé de détermination de la qualité d'un chaume,
**caractérisé en ce que**
le matériau chaume est incubé avec des champignons ligninolytiques d'une souche unique ou de plusieurs souches différentes et/ou avec des milieux conditionnés de tels champignons ou mélanges de champignons.

2. Procédé selon la revendication 1, comprenant les étapes suivantes :
a) préparation du matériau chaume ;
b) incubation du matériau chaume avec des champignons ligninolytiques d'une souche unique ou de plusieurs souches différentes et/ou avec des milieux conditionnés de tels champignons ou mélanges de champignons ;
c) détermination des modifications du matériau chaume incubé.

3. Procédé de contrôle de procédés de protection prophylactique d'un chaume, comprenant les étapes suivantes :
a) préparation du matériau chaume ;
b) traitement du matériau chaume avec un procédé à contrôler de protection prophylactique du chaume ;
c) incubation du matériau chaume avec des champignons ligninolytiques d'une souche unique ou de plusieurs souches différentes et/ou avec des milieux conditionnés de tels champignons ou mélanges de champignons ;
d) détermination des modifications du matériau chaume incubé.

4. Procédé selon une des revendications précédentes,
**caractérisé en ce que**
le matériau chaume est constitué d'un tronçon de tige d'une longueur de 0,1 cm à 50 cm de longueur, de préférence d'une longueur de 1,5 cm à 15 cm et qui provient également de préférence de la zone de tige proche de la racine et/ou qui ne présente pas d'entre-noeuds.

5. Procédé selon une des revendications précédentes,
**caractérisé en ce**
**qu'**on utilise des champignons ligninolytiques et/ou des milieux conditionnés de tels champignons ou mélanges de champignons qui présentent de la laccase et/ou une activité de la peroxydase.

6. Procédé selon une des revendications précédentes,
**caractérisé en ce**
**qu'**on utilise des champignons ligninolytiques qui proviennent du groupe des champignons de pourriture blanche et/ou des champignons à décomposition disséminante, de préférence des genres pycnoporus, trametes coprinus, geotrichum et/ou mycena, en particulier des espèces pycnoporus cinnabarinus, trametes versicolor et/ou coprinus domesticus et/ou de milieux conditionnés de tels champignons ou mélanges de champignons.

7. Procédé selon une des revendications précédentes,
**caractérisé en ce que**
des champignons ligninolytiques sont utilisés sous forme de composés d'inclusion de gel.

8. Procédé selon une des revendications précédentes,
**caractérisé en ce que**
la détermination de modification du matériau chaume incubé comprend la détermination d'une modification du poids sec, de la teneur en lignine, de la densité, de la dureté, de la résistance à la pénétration et/ou de la stabilité.

9. Procédé selon une des revendications précédentes,
**caractérisé en ce que**,
lors de la détermination de la modification du matériau chaume incubé, on utilise un appareil de mesure de la dureté dont le corps de contrôle peut être stérilisé.

10. Procédé selon une des revendications précédentes,
**caractérisé en ce que**
le matériau chaume est, de façon parallèle et/ou ensuite dans une étape supplémentaire, incubé avec des bactéries et/ou des champignons décomposant la cellulose ou l'hémicellulose.

11. Procédé selon une des revendications précédentes,
**caractérisé en ce que**
en plus, la teneur initiale en lignine, la teneur en azote et/ou la propriété hygroscopique du matériau chaume sont déterminées.

12. Utilisation de champignons ligninolytiques et/ou de milieux conditionnés de tels champignons ou mélanges de champignons pour la détermination de la qualité d'un chaume prévu pour la couverture de toitures.

13. Utilisation de champignons ligninolytiques et/ou de milieux conditionnés de tels champignons ou mélanges de champignons pour le contrôle de procédé de protection prophylactique du chaume.
